(19) Europäisches Patentamt
European Patent Office
Office européen des brevets

(11) **EP 1 173 097 B1**

(12) **EUROPEAN PATENT SPECIFICATION**

(45) Date of publication and mention
of the grant of the patent:
**23.07.2008 Bulletin 2008/30**

(51) Int Cl.:
***A61B 8/08*** (2006.01)

(21) Application number: **99915423.0**

(22) Date of filing: **16.04.1999**

(86) International application number:
**PCT/CA1999/000350**

(87) International publication number:
**WO 2000/062676 (26.10.2000 Gazette 2000/43)**

(54) **ULTRASONIC SCANNING APPARATUS**

ULTRASCHALL-ABTASTEINRICHTUNG

APPAREIL D'EXPLORATION A ULTRASON

(84) Designated Contracting States:
**AT BE CH CY DE DK ES FI FR GB GR IE IT LI LU MC NL PT SE**

(43) Date of publication of application:
**23.01.2002 Bulletin 2002/04**

(73) Proprietor: **Texon Technologies Ltd.
Vancouver,
British Columbia V6C 1H2 (CA)**

(72) Inventor: **KOBLANSKI, John
Burnaby, British Columbia V5H 1K2 (CA)**

(74) Representative: **Ebner von Eschenbach, Jennifer et al
Ladas & Parry LLP
Dachauerstrasse 37
80335 München (DE)**

(56) References cited:
**EP-A- 0 761 169        EP-A- 0 786 232
US-A- 4 276 491         US-A- 5 003 965
US-A- 5 335 661**

**Description**

This invention relates to an apparatus and to a method to measure the physical characteristics of an object. The apparatus and method find particular application measuring the physical characteristics of bone and is thus of value in diagnosing osteoporosis and osteomalacia. However the invention finds general application in assessing the structural properties of materials and in detecting minute changes in that structure. Although this is of particular value in determining the integrity of heterogenous materials such as bone, the invention, in both aspects, is of wider application.

BACKGROUND OF THE INVENTION

Osteoporosis is a condition, more common amongst women than men, characterized by deterioration of the bone. The bone becomes porous and brittle. Osteoporosis at present is diagnosed by measuring the density and elasticity of the bone. High density alone does not determine the bone resistance to fracture. The bone can possess quite high density but still be brittle and therefore susceptible to breakage. Prior art methods of measuring bone density do not have the required degree of accuracy to determine small changes in bone density, which is what is needed to establish optimum therapeutic or diagnostic procedures.

Osteomalacia is a condition in which softening of the bone occurs. Softening is the result of absorption of calcium from the bones. It occurs especially in pregnant women and it is believed to be related to a dietary deficiency in vitamin D.

My co-pending United States Patent Application Serial No. 018,709 filed February 17, 1993 describes and claims an apparatus to measure the physical characteristic of an object. The apparatus has a bath to receive the object and the object can be stabilized in the bath. Liquid is supplied to and from the bath. The temperature of the liquid can be controlled so that it is above the temperature of the object. An ultrasonic transmitter sends a signal through the object and an ultrasonic receiver receives the signal. The velocity of the signal of the object can be calculated. This apparatus is useful in diagnosing osteoporosis.

The heel bone is suited to ultrasonic measurement with its sides relatively parallel. The heel bone is composed mainly of trabecular bone. The elasticity and strength of this bone is provided by its sheet structure. The manner in which trabeculae are assembled is more significant than the volume or any other characteristic of bone. Rigidity and strength is more a matter of geometry than mass. The trabeculae arrangement, and the contiguity that it provides, is a more important parameter than volume or weight for action of hard tissue in determining the stiffness of trabecular bone. Because of these facts the known methods of utilizing the velocity of sound in bone and the attenuation measurements, although appearing to provide sound theoretical methods, have not been satisfactory in diagnosing osteoporosis and are not capable of evaluating the effectiveness of any treatment procedure.

Another difficulty with existing methods is their dependence upon references or standards. Using the standards creates an additive error to any measurement. In addition to this error, the use of standards having homogenous composition is undesirable as these compositions relate poorly to human bone; human bone is a heterogenous material.

X-ray methods have been used to diagnose osteoporosis but have also been found unsatisfactory. They measure only the density and provide no quantitative evaluation of structure. Because they produce ionizing radiation, X-rays are not suitable for this purpose.

No prior art has shown an ability to measure bone structure, density and the velocity of sound in bone using a single device. The use of a single device provides a means of early detection as well as a more accurate assessment of the degree of osteoporosis.

All the present methods depend highly on instrument electronic stability. This can be undesirable. There can also be a lack of uniformity in the transducer characteristic, stemming from the manufacturing process.

Furthermore the accuracy with which the heel can be re-positioned for repeat measurements is not particularly satisfactory in the prior art. The reapplying of the transducer against the heel with the same conditions, particularly the same contact pressure, for subsequent measurements is also not well done in the prior art.

SUMMARY OF THE INVENTION

The present invention seeks to avoid the disadvantages in the prior art. In particular the present invention allows for instrumental base line drift without the introduction of errors into the measurement. The invention also provides for extremely accurate location of the heel and easy repetition of the location of the heel for subsequent measurements.

A particular advantage of the present invention is that it is self-calibrating.

Accordingly, the present invention is an apparatus according to claim 1 and a method according to claim 21. Preferred embodiments of the invention are defined in the dependent claims.

BRIEF DESCRIPTION OF THE DRAWINGS

The invention is illustrated in the drawings in which:

Figure 1 is a plan view of an apparatus according to the present invention;
Figure 1a is a detail of Figure 1;
Figure 2 is a side elevation of the apparatus of Figure 1;
Figures 3a and 3b are further details of Figures 1 and 2;
Figure 4a is a detail of an ultrasonic transmitter useful with the apparatus of the present invention;
Figure 4b is a detail view of an ultrasonic receiver for use with the apparatus of the present invention;
Figure 5 shows schematically the apparatus for the present invention;
Figure 6 illustrates the computer and control circuitry with signal transmission and processing; and
Figures 7a, 7b and 7c are general views;

DESCRIPTION OF THE PREFERRED EMBODIMENTS

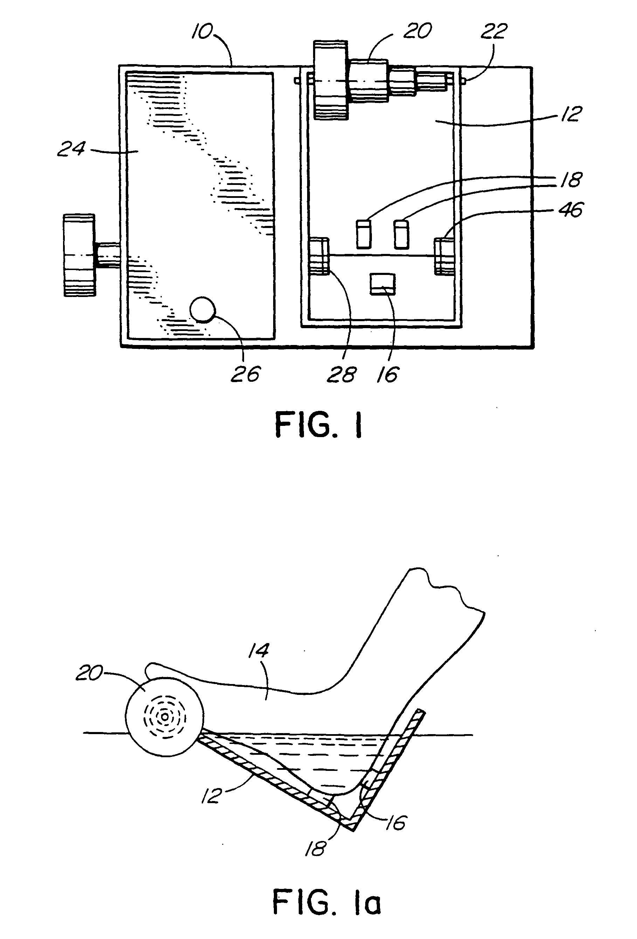

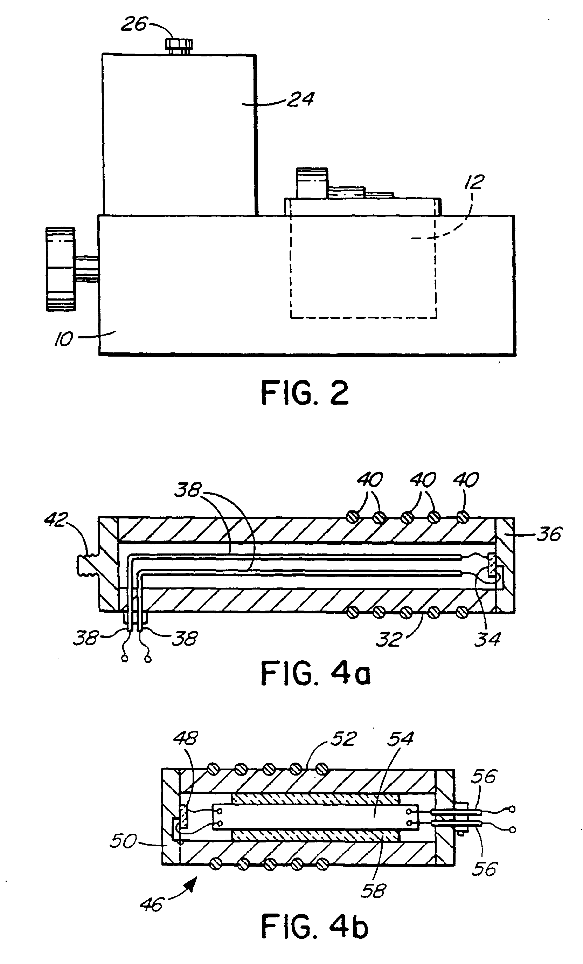

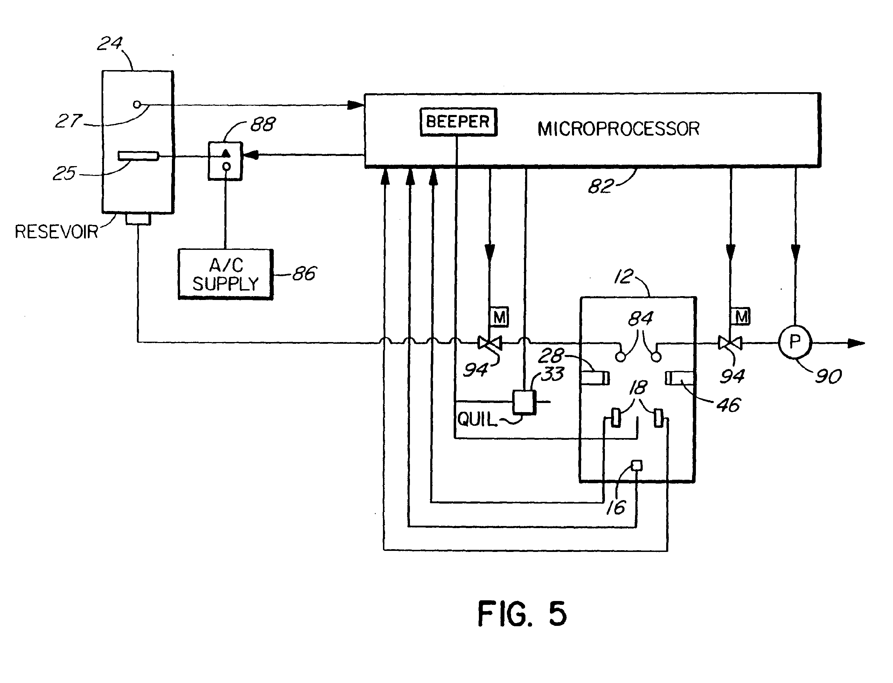

The drawings show an apparatus to measure the physical characteristics of an object. As shown particularly in Figures 1 and 2 the apparatus has a main body 10 that includes a recess 12 to receive the object 14, typically a foot as shown in Figure 1a. The apparatus includes means to determine when the object is correctly positioned in the recess. In the illustrated embodiment that means comprises a plurality of pressure sensors. There is a pressure receptor 16 to contact the heel, as shown in Figure 1a, and a pair of pressure sensors 18 to contact under the heel, again as shown in Figure 1a. The operation of these sensors is discussed subsequently, notably with regard to Figure 5.

In addition the apparatus of Figure 1 includes a toe stabilizer 20 also useful to determine the correct position of the object in the recess when the object is a foot 14. The toe stabilizer 20 is stepped and is received in that part of the recess 12 remote from the pressure sensors 16 and 18 for the heel of the foot. As shown particularly in Figure 1a the big toe of the user is placed against the toe stabilizer and the position thus achieved. It should be noted that the stabilizer is removably attached in the recess which means it can be used for either the left or the right foot. To facilitate removal stabilizer 20 includes a spring loaded mounting 22 shown in Figure 1.

There is a reservoir 24, having a filling cap 26, to hold liquid to be used in the recess 12 during measurement. In this regard, however, it should be emphasized that the use of a fluid and, indeed, the use of a recess 12 to receive the fluid is not essential to the apparatus of the present invention. A virtue of the equipment, at least compared with applicant's own prior art, is that the fluid reservoir 24, the recess 12, and the heating of the fluid are not necessary in the apparatus of the present invention. A viscous liquid or gel may be interposed between the heel and the transducer. Other applications may necessitate the total immersion of the object to be measured, for example if the object has irregular surfaces.

The apparatus of the present invention as illustrated includes heating means 25 (see Figure 5) to ensure that the liquid contained in the reservoir 24, and thus in the recess 12 during measurement, can be maintained at a temperature slightly above the temperature of the object being assessed. This prevents the formation of bubbles in liquids like water, which interfere with the reflecting ultrasound. Temperature is measured by a sensor 27.

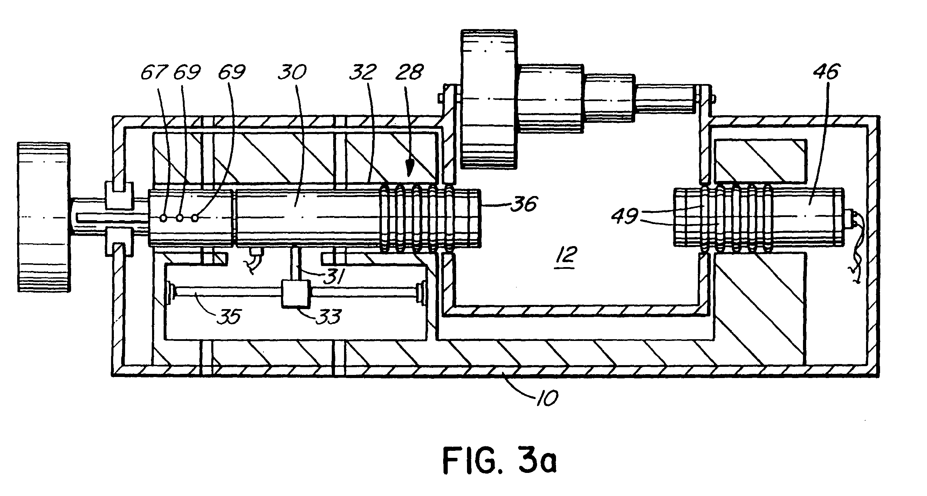

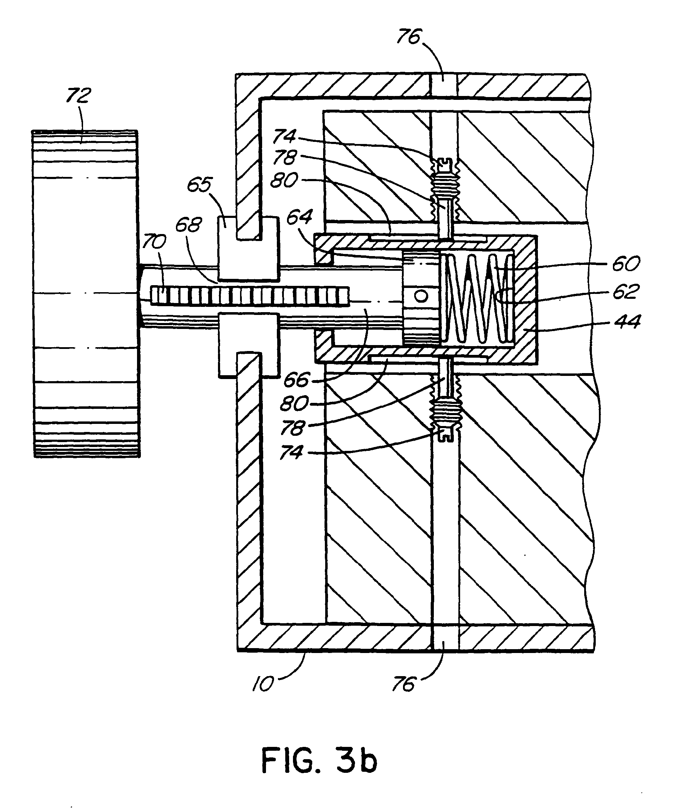

There is a piezoelectric ultrasonic transmitter 28 to send a signal through the object 14. As shown particularly in Figures 3a, 3b and 4a the ultrasonic transmitter 28 comprises a first, hollow container 30 that is slidably received in a recess 32 in the main body 10. There is a ceramic transmitter 34, shown in Figure 4, attached to a face plate 36 of the container 30. The container 32 includes connections 38 that extend to a pulse amplifier discussed below.

In addition the container 32 includes O-rings 40 located on its exterior to seal and also to facilitate movement of the container 32 within the main body 10. At its trailing end there is a stud 42 that allows attachment of the container 32 to a spring chamber 44, shown in Figure 3b.

The apparatus also includes a piezoelectric ultrasonic receiver 46, shown particularly in Figure 3a and 4b, and spaced from the transmitter 28 across the recess 12. Like the container 30 of the transmitter 28 the receiver 46 also has O-rings 49 on its outer surface. The ultrasonic receiver 46 including transducer 48 mounted on an end plate 50 of a cylinder 52. O-rings 49 are around the exterior of the cylinder 52 and there is a preamplifier 54, attached to a micro processor through connection 56. There is also an insulating spacer 58 within the second cylinder 52.

Container 30 has a bolt 31 extending from it. A quill 33 is attached to bolt 31 and moves along an optical ruler 35. This is used to determine the position of the container 30 and thus of the transmitter 28.

In both the transmitter and the receiver, the face plates 36 and 50 are desirably resilient and the piezoelectric ultrasonic receiver and transmitter 34 and 48 are ceramic transducers, resiliently mounted. In a preferred embodiment the face plates 36 and 50 are of an adhesive sealant which functions as a resilient seal for the cylinder 32 and 52 and also acts as a supporting structure for the piezoelectric ceramics. In a preferred embodiment the face plates 36 and 50 also act as focusing devices for the equipment.

The ultrasonic transmitter 28 is urged into the recess 12 in the main body 10 by the provision of spring chamber 44 shown in Figure 3b and including an internal spring 60. Spring chamber 44 has a threaded opening 62 that receives the stud 42 shown in Figure 4. The spring 60 abuts an end 62 of the third chamber 44 and a piston 64, mounted on a rod 66 that extends out of the third chamber 44. The piston acts to compress the spring 60. There are means to fix the position of the piston 64 and thus the tension of the spring 60 in the form of screws 67 that are received in holes 69 as best shown in Figure 3a. In an alternative arrangement illustrated in Figure 3b, there is collar 65 mounted in the main body 10, and provided with engagement means, typically in the form of a recess 68. There are projections or engaging gears 70 on the rod 66 able to engage with the recess 68 in the collar 65. The rod 66 also has a handle 72 to allow rotation.

Using this equipment the rod 66 may be pushed inwardly, until the appropriate tension is achieved in the spring 60 and then rotated so that the engagement means 65 and 68 engage each other to fix the position of the piston 64 and thus the tension in the spring 60.

The tension of the spring 60 can be pre-set by the use of screws 74 that can be located through passageways 76 to be screwed inwardly to compress a nylon bearing 78 into channels 80 provided in the cylinder 44.

Figure 5 shows the control of the equipment. There is a microprocessor 82 that receives signals from the pressure sensors 16 and 18 and acts to control filling and empty of the recess 12 through ports 84 and the temperature of the fluid in the recess. There is an alternating current supply 86 and an on/off relay 88 controlling the equipment.

The microprocessor 82 will ensure that the recess 12 is filled and emptied, using a pump 90 and the motorized valve 94 shown in Figures 5, according to a pre-set plan. The microprocessor 82 will ensure that the recess 12 will not be filled with liquid, usually water, at an undesirable temperature and will also ensure that the recess 12 is filled and drained as appropriate. It will also ensure that readings cannot be taken unless the heel is properly located. This is done by scanning the signals from the pressure sensors 16 and 18.

Information from the quill 33 is also stored in microprocessor 82.

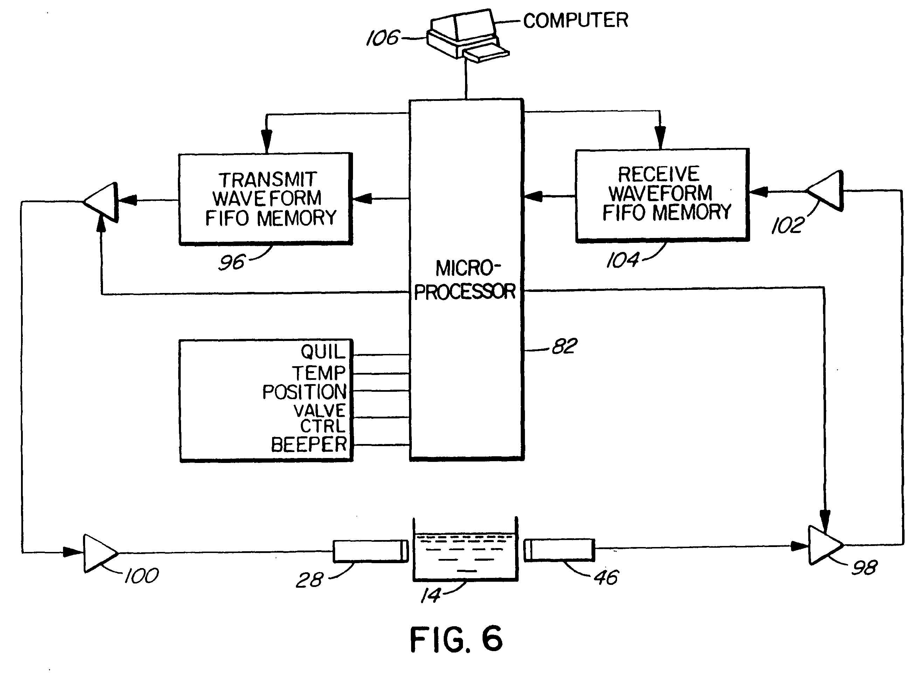

Figure 6 illustrates the control circuitry. The microprocessor 82 generates a digital representation of the desired transmit waveform into a transmit FiFo (first in first out) memory 96. The microprocessor 82 sets the gain of transmit digital/analog (D/A) and the gain at a receiver pre-amplifier 98. The microprocessor initiates wave form transmission from transmit Fifo 96 through the video D/A power amplifier 100 and the transmit transducer 28. Sound energy from the transmitter 28, having passed through the heel of foot-14, is detected by the receiving transducer 46 and the signal is amplified by the programmable gain pre-amplifier 98 digitized by the video A/D converter 102 and stored in the receiver FiFo memory 104. The microprocessor 82 then retrieves the received waveform from the receiver FiFo memory 104 and performs an analysis. The microprocessor 82 then sends the receive waveform for analysis to a personal computer 106 for storage and display.

To operate the equipment of the present invention as illustrated in the drawings, the microprocessor is programmed to automatically control the temperature, to fill up the empty bath, to record patient names and other data. All of this information is controlled as shown in Figure 6. This is shown schematically as this software is well known.

The reservoir 24 is filled and the temperature in the reservoir raised sufficiently to yield a temperature at several degrees higher in the recess than the temperature of the object, typically a foot. The patient's foot is cleansed with detergent, rinsed and thoroughly dried. A small amount of a wetting agent is added to the reservoir. The heel is then placed in the bath 12 so that specified pressure is exerted against the back and sole part of the heel and this information relayed to the microprocessor by the pressure sensors 16 and 18. If the sole pressures, measured by the sole sensor 18 shown in Figure 5, are unequal the knee is moved to the left or right to achieve equal pressure. This aligns the heel bone perpendicular to the transducers. The knee is maintained in the same position for the duration of the test.

Knob 72 with the off position showing vertically, is pushed until the preset contact pressure against the heel is achieved. The knob 72 is then rotated until the on position is vertical, that is to say the rod 66 is locked within the collar. This enables the retainer screws to hold the spring 60 in a compressed position. The receiver piezoelectric element and associated mechanical mounting allow the receiver to vibrate freely with a minimum of energy lost to the casing. In this configuration the transducers exhibit two strong resonant frequencies, 171,400 Hz and 668,400 Hz. The predominant receiver frequency is the former 171,400 Hz. The effect of these freely resonating frequencies is that when the receiver is impacted with a small amount of ultrasonic energy, or at the end of receiving a large amount of ultrasonic energy, the receiver exhibits a strong tendency to vibrate 171,400 Hz. The strength of this tendency is directly dependent upon a constant which is the modulus of elasticity of the ceramic material. Thus the material does not need calibration or reference standard. These receiver characteristics and resonant frequencies are used to advantage in determining bone integrity in the following way.

If the transmitter produces, say, two cycles (sinusoidal) of ultrasound at 668,400 Hz the receiver will begin vibrating at 668,400 Hz and then transition to vibrating at 171,400 Hz will take place until the vibrations end. The energy expended by the receiver vibrating at 668,400 Hz versus the energy vibrating at 171,400 Hz depends on the amplitude of the received ultrasound which, in turn, depends on the energy absorbed by the heel. When the signal arrives at the receiver the microprocessor performs a predetermined gain adjustment on the programmable gain preamplifier according to the scheme of Figure 6. This produces a trace of sufficient magnitude for accurate analysis. As the amplitude of the trace for analysis is thus increased or decreased to a predetermined amplitude, it can be said that it is independent of the density or thickness of the heel bone. Therefore it is known that as humans age they lose density. However the loss of structure is a serious event that could lead to fracture. Thus in this method good bone quality could have the same value at 65 as at 25 years of age, unlike bone density, which decreases with age.

A useful measure of bone integrity has been achieved by spectral analysis of the above received waveform between 80 KHz and 245 KHz, the low frequency and between 245 KHz and 860 KHz for the high frequency component and forming a % ratio according to the formula:

$$\text{T-index} = \frac{[\text{Area of High Frequency Component} \times 100]}{\text{Area of Entire trace}} - 100 = \%.$$

This equation yields a new type of index, termed the trabecular or t-index of osteoporosis that is easily interpreted. In general a t-index value of 80% to 90% represents healthy bone. Decreasing values below 80% correlate with increasing structural problems. Values as low as 30% have been found in bone having structural problems leading to fracture. As the structural integrity is measured this test can differentiate between osteomalacia and osteoporosis as the integrity of the structure is affected in the latter but not in the former.

An alternative method according to the present invention eliminates the need for spectral analysis. According to this alternative method the transmitter produces, say, a 2 cycle Sinewave burst of ultrasound at 668,400 Hz and the trace is recorded. A second trace of a single cycle is generated at 171,400 Hz and the amplitude adjusted so that the received waveform is the same amplitude as the original waveform. Now the two waveforms can be matched in time so that a minimum difference waveform is obtained. The area of the difference of the waveform is the high frequency component in the ratio above and the area of the second waveform is a total area of the same ratio. This method eliminates the complex spectral analysis and attains a higher lever of accuracy.

In addition to providing a structured integrated value, the apparatus of the present invention can measure bone density and the velocity of sound in bone. Although the density and velocity do not detect the initial stages of osteoporosis they do provide an indication of how advanced the disease is. Unlike the trabecular (structural) index measurement, the density and velocity measurements require a heel width measurement. The quill 33 shown in the drawings records the horizontal movement of the transmitting transducer 28. The quill 33 is zeroed by inserting a known width gauge between the transducers 28 and 46. This width is inserted into the program and added to subsequent measurements by the quill 33.

The rationale for measuring bone density is as follows:

It has been observed that the low frequency wave (say 171400Hz) passes through the heel bone with very little difference in attenuation from person to person. Whereas a much higher frequency wave (say 668,400Hz) shows large attenuation differences from person to person. These observations enable a self-calibrating method for measuring bone density as follows. A number of measurements are performed on a single individual preferably a healthy male. These measurements consist of recording the voltages necessary to transmit at a low frequency (171,400 Hz) and obtain 180 units of amplitude on the recorder. The lowest value obtained represents optimum coupling between the transducers and the heel. This voltage value is inserted into the program and is referred to as the low frequency normalization factor (L.F.N.F.) and used in the density measurements for all patients.

In operation the heel is maintained in position and the voltages needed to achieve 180 units at the recorder for the low and high frequency pulse (171,400 Hz) are increased or decreased by the microprocessor and the calculation of density computed as follows:

$$\text{Density} = \frac{\text{L.F.N.F. Amplitude (volt)}}{\text{Low Frequency Amplitude (volts)}} \times \frac{\text{High Frequency Amplitude (volt)}}{\text{Heel Width (mm)}}$$

$$\text{Density} = \text{Volts/Millimetre (Units)}$$

$$\text{Density} = \% \text{ of normal young adult value} = \frac{\text{Patient Value}}{\text{Normal Young Adult Value}} \times 100$$

The present device measures bone density more accurately than previous devices because no phantom (reference standard) is needed. Further the degree of coupling between the test object and the transducers is not a source of error as the L.F.N.F. makes a correction for this.

The position of the foot is maintained for the velocity measurement. By recording the time interval for the pulse to travel through the heel and dividing the heel width by this time the velocity is determined in meters/second. The high frequency trace (668,400 Hz) is used to note the time of arrival of the pulse. This is difficult for the computer to detect as the beginning of the trace is ill-defined. The trace is amplified by the preamplifier enabling the beginning of the trace to be detected by the human eye. The computer easily measures the time of second crossing of the base line as displayed on a monitor. That time interval is measured and has been found to be relatively constant for all patients at 2.026 microseconds. As the computer can easily detect the second crossing of the baseline of the trace used for analysis the above constant value of 2.026 is subtracted from the value to provide the precise time of arrival of the pulse. Other than this unique feature of ensuring that the time is measured with extreme accuracy, the velocity method and its use in evaluating bone is well documented and will not be further elaborated on.

Unlike any other device, this method provides the measurement of three parameters of bone quality, namely structural integrity, bone density and velocity of sound in bone. This enables early detection of osteoporosis and evaluates the therapeutic procedures to correct the condition.

Although the invention describes particularly the use of the method and apparatus to diagnose osteoporosis the apparatus and method can be directly applied to process control in the process industries - food, pulp and paper, petrochemical, pharmaceutical, paint, dairy etc.

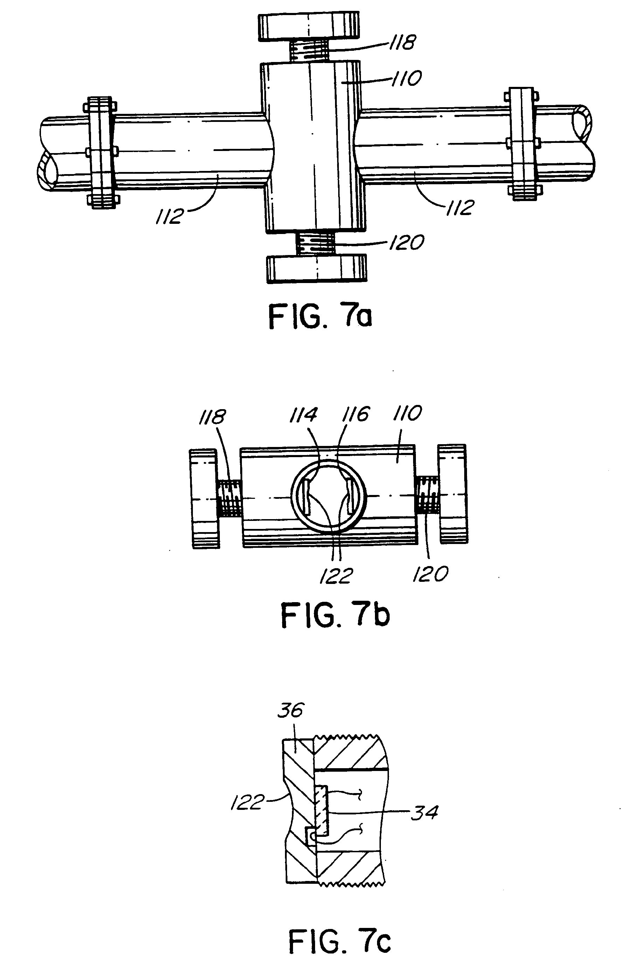

Examples - Pulp and Paper Industry - The "t-index" above may be used to indicate the amount of water in the pulp when flowing or in a fixed sample (See Fig. 7). The higher the pulp to water ratio the higher will be the "t-index".

Example - Solids content in the Food Industry - The higher the particulate in a liquid the higher the "t-index".

Example - Density Measurements - The value of density measurements throughout industry is well documented. However, the ultrasonic methods used are not as accurate as the method of the present invention.

In other applications, a higher intensity of sound may be needed. In this case the transducer face plate of adhesive sealant is shaped to achieve the desired focusing of the sound beam as shown in Fig. 7. If the higher intensity is insufficient the receiving and transmitting transducers may be brought closer together by rotating them clockwise, assuming the body of the transducer has a right hand thread. In all applications two resonating frequencies are utilized - a higher and a lower frequency achieved by having piezoelectric ceramics operating in the thickness and radial mode and mounted so as to minimize the damping.

Although the above method and apparatus are described particularly in relation to diagnosis of osteoporosis it can also be used to distinguish between osteomalacia and osteoporosis and can also be directly applied to process control in industry, for example in the food, pulp and paper, petro chemical, pharmaceutical, paint and dairy industries. Assuming the application is pulp and paper, one application in this industry is to determine the amount of water in a slurry of pulp. The higher the pulp to water ratio the higher will be the index as discussed above. Both the spectral analysis and the different curve will provide the information.

Apparatus to enable the determination of this is shown in Figure 7 where a simple container 110 is installed in a pipeline 112 in which a transmitter 114 and a receiver 116 are mounted on threaded, sealed member. The necessary signals are taken and interpreted as shown in Figure 6. The relevant equipment is not shown in Figure 7. Here is a particular example of the face plates acting as focusing members and to that end they are provided with concave recesses 122.

In other applications a high intensity of sound may be needed. In this case a transducer face plate is shaped to achieve the desired focusing of the sound, as shown in Figure 7b. If the high intensity is not attained the receiving and transmitting transducers may be brought closer together by rotating them clockwise on the threads. The resonating frequencies are always used - and a higher and a lower frequency by having the ceramics operate in the thickness and radial mode respectively.

This aspect of the invention is of extreme importance. For efficient transfer of power from the generator to the medium the two must be acoustically matched. The specific impedance of the ceramic is approximately 30 $g \cdot s^{-1} \cdot m^{-2}$ (30mRayls) and that of water is 1.5 $g \cdot s^{-1} \cdot m^{-2}$ (1.5 mRayls), there is a need to smooth out the discontinuity so that the transfer of energy from the transducers to the medium is maximized. A layer of a material with an acoustics impedance intermediate between the ceramic and water, by being interposed between the two, provides good matching using polymers with impedances of about 3.5 $g \cdot s^{-1} \cdot m^{-2}$ (3.5mRayls). These are readably available. The velocity of sound in these are approximately 2400 metres per second so the thickness required will vary with the frequency used.

Both transducers are air backed. The transmitter is made of a material of 5400 Navy (U. S. A.) whereas the receiver is 5500 Navy (U. S. A.). The diameters of both on 1.25 cm (0.5 inches) and the thicknesses of both are 0.25 cm (0.1 inches). The main body of the transducers should have an outside diameter of about 2.5 cm (1 inch) and an inner diameter of about 1.5 cm (0.6 inches). As the ceramic is only 1.25 cm (0.5 inches) compression of the ceramic between the body and the object to be measured is prevented. It is only the face plate that is compressed between the object and the main body. The characteristic of the sealant is such that it possesses strong adhesion and remains pliable and is a non-conductor of electrical current. A preferred material for this use is that available under the trademark E 6000 from Eclectic Products, which is a styrene based adhesive sealant. The combined effect of the sealant and the transducer ceramic geometry, that is 5 to 1 ratio of diameter to thickness, disc shape and ceramic mounting techniques allow *both transducers* to resonate in both the radial and thickness modes. This provides a low resonating frequency in the radial mode and a high resonating frequency in the thickness mode of vibration. This method of mounting ceramic and using a compliant or resilient face plate not only produces a highly efficient transducer but enables production of piezoelectric transducers that have virtually the same characteristics. The geometry of the transducers is such that the radial mode is preferred. That is if a single burst of energy, of the higher resonating frequency is received by the receiver it will start vibrating at this higher frequency, in the thickness mode and change into the radial mode, proportional to the energy of the burst received. That is to say the length of time spent in the thickness mode is proportional to the energy of the burst received. This competition between radial and thickness modes of vibration is based upon the modulus of elasticity of the ceramic and provides its own reference as the modulus of elasticity is a constant, thus avoiding the necessity for calibration.

Although the present invention has been described in some detail by way of example for purposes of clarity and understanding, it will be apparent that certain changes and modifications may be practised within the scope of the appended claims.

**Claims**

1. Apparatus to measure the physical characteristics of an object comprising a transmitting piezoelectric first transducer (28; 34) and a receiving piezoelectric second transducer (46; 48) to receive energy from the first transducer, said first (28; 34) transducer and second (46; 48) transducer being spaced apart to receive the object between them; the apparatus being **characterized in that** the first (34) and second (48) transducers are each mounted on a resilient face plate (36, 50) by one face, whereby each of the transducers is able to resonate radially and axially upon receipt of an energy pulse.

2. Apparatus as claimed in claim 1 including means (38; 82, 96, 100) to apply voltage to the first transducer (34; 28) and means (54, 56, 58: 82, 99,102, 104) to measure the energy in the second transducer (48; 46).

3. Apparatus as claimed in claim. 1 in which the first (34) and second (48) transducers are each mounted within a tube (32, 52) having a face plate (36, 50) at its end.

4. Apparatus as claimed in claim 3 in which each face (36, 50) plate is molded to a predetermined shape (122) to focus radiation.

5. Apparatus as claimed in claim 1 in which the resilient faceplate (36, 50) has the following characteristics:

   (i) strong adhesion to the face of the ceramic and to the cylindrical main body;
   (ii) acoustic impedance between that of a ceramic and human tissue;
   (iii) inert to caustics, acids and oils;
   (iv) mouldable; and
   (v) electrically non-conductive.

6. Apparatus as claimed in claim 5 in which the resilient material is a styrene based polymer.

7. Apparatus according to claim 1, further comprising;
   a main body (10) to receive the object (14);
   means (16, 18, 20) to determine when the object is correctly on the body;
   the transmitting piezoelectric first transducer (28) being a piezoelectric ultrasonic transmitter to send a signal through the object, said transmitter comprising a first hollow container (32) that is slidably received in the main body (10) and a piezoelectric ceramic transmitter (34) resiliently mounted in said first container (32), said transmitter having a diameter about 5 times greater than its thickness;
   means (44, 60) urging said transmitter (28) towards said object;
   means (67, 69) to restrict the movement of said transmitter (28);
   the receiving piezoelectric second transducer (46) being a piezoelectric ultrasonic receiver spaced from said transmitter and also projecting towards said body, said receiver comprising a second hollow container (52) slidably received in said main body (10), and a ceramic transducer (48) resiliently mounted in said second hollow container (52), said transducer having a diameter above 5 times greater than its thickness;
   means (38) to generate a signal to be transmitted; and
   means (54) to amplify said received signal.

8. Apparatus as claimed in claim 7 in which the object (14) is a foot.

9. Apparatus as claimed in claim 7 in which the means (16, 18, 20) to determine when the object is correctly positioned comprises:

   a first pressure sensor (18) to contact the under side of the object; and
   a second pressure sensor (16) to contact the rear of the object; and
   means (82) to generate a signal when pressure is correctly applied to the first and second sensors.

10. Apparatus as claimed in claim 9 in which there are two first sensors (18).

11. Apparatus as claimed in claim 9 in which, when the object (14) is a foot, the means (16, 18, 20) to determine when the object is correctly positioned includes a toe stabilizer (20) mounted in said recess to abut a side of the toe to ensure the foot is in the correct position.

12. Apparatus as claimed in claim 11 in which the toe stabilizer (20) is stepped for variable size feet.

13. Apparatus as claimed an claim 11 in which the toe stabilizer (20) is reversible so that it can be used with both left and right feet.

14. Apparatus as claimed in claim 7 in which said ultrasonic transmitter (28) includes means (100) to amplify a pulse.

15. Apparatus as claimed in claim 7 including O rings (40, 49) on said first and second hollow containers to allow the hollow container to slide in said main body (10).

16. Apparatus as claimed in claim 7 in which the ceramic transducers (34, 48) are mounted on a resilient end plate (36, 50) of said first and second bodies (32, 52).

17. Apparatus as claimed in claim 16 in which said end plates (36, 50) are formed of a styrene based resilient adhesive.

18. Apparatus as claimed in claim 7 in which the means (44, 60) urging said transmitter (28) into said recess comprise a spring (60) mounted in a third container (44), attached to first container.

19. Apparatus as claimed in claim 18 in which the spring (60) abuts an end of said third container (32);
   a piston (64) to abut said spring to compress said spring;
   means (67, 69) to fix the position of the piston (64) and thus the tension of the spring (60).

20. Apparatus as claimed in claim 19 in which the piston (64) is mounted on a rod (66) that extends out of said third container (44) and out of said main body (10);
   a collar (65) on said main body having first engagement means (68);
   a second engagement means (70) on said rod (66), engageable with said first engagement means (68) whereby rotation of the rod (66) acts to release and engage said first and second engagement means,

21. A method of measuring the physical characteristics of an object comprising:

   receiving energy from a transmitting first piezoelectric transducer in a second receiving piezoelectric transducer, wherein the first piezoelectric transducer and the second piezoelectric transducer are spaced apart to receive the object between them;
   the method being **characterized in that** said receiving energy from a transmitting first piezoelectric transducer in a second receiving piezoelectric transducer comprises receiving energy from a transmitting first piezoelectric transducer in a second receiving piezoelectric transducer, wherein the first (34, 48) and second piezoelectric transducers are each mounted on a resilient face plate (36, 50) by one face, whereby each of the piezoelectric transducers is able to resonate radially and axially upon receipt of an energy pulse.

22. The method of claim 21, comprising subjecting the object to resonating frequencies from the first piezoelectric ultrasonic transmitter, a first resonating Frequency obtained by resonating in the thickness mode and a second, lower resonating frequency, obtained by resonating in the radial mode.

23. A method as claimed in claim 22 that comprises determining the optimum high and low frequencies by finding a low frequency component having low attenuation on passage through the object and finding a high frequency component having high attenuation on passage through the object.

24. The method of claim 21, comprising;
submitting the object to a high frequency burst using both a radial and thickness mode resonance of a piezoelectric transducer transmitter;
measuring the energy transmitted through the object by said high frequency burst; and
expressing the energy as a percentage of a total energy received that comprises:

(i) recording the voltage trace outputted by the receiver;
(ii) analyzing the trace by spectral analysis or difference trace;
(iii) deriving an expression:

$$\frac{[\text{area of high frequency component}]}{[\text{area of entire trace}]} \quad \begin{array}{l} \times\, 100 \\ -\, 100 \end{array}$$

said expression determining the percentage transmission to the higher frequency component without the need to use a reference.

25. A method as claimed in claim 24 for deriving an index of structural integrity of bone by using the expression defined in claim 25, a high percentage indicating good structure and values below 80% indicating progressively poorer bone.

26. The method of claim 21, wherein the object is bone, and comprising

(i) measuring bone density using radial and thickness modes of resonance of a piezoelectric transducer transmitter without the use of a reference by eliminating error due to variability in the coupling medium,
(ii) determining the minimum voltage of a low frequency burst displaying negligible attenuation on passage through the bone to achieve the amplitude to be used for future measurements by using a large number of measurements on a normal subject to find the minimal voltage, said minimal voltage being a normalizing factor (LFNF) used on all subsequent measurements of all subjects according to the relationship:

$$\text{Density} = \frac{\text{LFNF (voltage)}}{\text{low frequency (voltage)}} \times \frac{\text{High frequency (voltage)}}{\text{Heel width (mm)}}$$

$$= \frac{\text{voltage}}{\text{mm}}$$

(iii) the density expressed as volts/mm, being determined on a large number of young adults according to the expression:

$$\text{Density} = \frac{[\text{patient (volt/mm) x100}]}{[\text{normal young adults (volts/mm)}]}$$

whereby density = a percentage of normal young adults.

27. The method of claim 21, comprising
using radial and thickness modes of resonance of a piezoelectric transducer to eliminate error due to a coupling medium by transmitting a pulse of low frequency and negligible attenuation through the object to obtain a desired fixed voltage at the receiver;
repeating these measurements on the same individual over a long period using the same coupling throughout and recording the lowest voltage needed at the transmitter/transducer to achieve the fixed voltage;
using this frequency voltage as a low frequency normalizing factor (LFNF);
sending a low frequency burst followed by a high frequency burst; and
expressing the LFNF as the numerator and the low frequency as the denominator to normalize the high frequency component.

28. The method of claim 21, comprising measuring the relative energy of a high frequency burst transmitted through the object by:

recording the received energy using a piezoelectric ceramic transducer having two modes of operation, namely a thickness mode of operation and a radial mode of operation;
performing a spectral analysis of a trace of the received energy;
determining the area of the high frequency component; and
comparing the area with that transmitted through a standard object of known characteristics, to show differences in composition or structure between the object and the standard.

29. A method as claimed in claim 28 in which the modulus of elasticity of the ceramic transducer is used to eliminate the need of external reference to measure the received energy.

30. The method of claim 21, comprising measuring the time of travel of a sound wave through the object, wherein the transmitting transducer is a highly undamped piezoelectric ceramic ultrasonic transducer operating in radial and thickness modes, by:

amplifying a trace of the sound wave;
measuring the time of first crossing of the base line of a trace of the received sound wave;
measuring a large number of similar objects to derive an average value and thus a constant time interval;
inserting this average value into a microprocessor for subtraction from the time of the second crossing of the base line as determined by the microprocessor; and
thereby obtaining the time of travel of the sound wave through an object,

31. A method as claimed in claim 30 in which the time constant is determined at 668,400 Hz as 2.026 microseconds for the human heel bone.

**Patentansprüche**

1. Vorrichtung zum Messen der physikalischen Eigenschaften eines Objekts, welche einen sendenden piezoelektrischen ersten Messwandler (28; 34) umfasst und einen empfangenden piezoelektrischen zweiten Messwandler (46; 48) für den Empfang von Energie von dem ersten Messwandler, wobei der genannte erste (28; 34) Messwandler und der zweite (46; 48) Messwandler räumlich voneinander getrennt sind, um das Objekt dazwischen zu empfangen; wobei die Vorrichtung **dadurch gekennzeichnet ist, dass** die ersten (34) und zweiten (48) Messwandler jeweils durch eine Seite an einer federnden Frontplatte (36, 50) angebracht sind, wodurch jeder der Messwandler in der Lage ist, bei Empfang eines Energieimpulses radial und axial in Resonanz zu treten.

2. Vorrichtung nach Anspruch 1, mit einer Einrichtung (38; 82, 96, 100) zum Anlegen einer Spannung an den ersten Messwandler (34; 28) und einer Einrichtung (54, 56, 58; 82, 89, 102,104) zum Messen der Energie in dem zweiten Messwandler (48; 56).

3. Vorrichtung nach Anspruch 1, wobei die ersten (34) und zweiten (48) Messwandler jeweils in einer Röhre (32, 52) angebracht sind, mit einer Frontplatte (36, 50) an deren Ende.

4. Vorrichtung nach Anspruch 3, wobei jede Frontplatte (36, 50) in einer vorbestimmten Form (122) geformt ist, um Strahlung zu fokussieren.

5. Vorrichtung nach Anspruch 1, wobei die federnde Frontplatte (36, 50) die folgenden Eigenschaften aufweist:

(i) eine starke Adhäsion an der Front des Keramikwerkstoffs und an dem zylindrischen Hauptkörper;
(ii) eine akustische Impedanz zwischen der eines Keramikwerkstoffs und der von menschlichem Gewebe;
(iii) inert ist gegen Beizmittel, Säuren und Öle;
(iv) formbar ist; und
(v) elektrisch nicht leitfähig ist.

**6.** Vorrichtung nach Anspruch 5, wobei es sich bei dem federnden Material um ein Polymer auf Styrolbasis handelt.

**7.** Vorrichtung nach Anspruch 1, wobei die Vorrichtung ferner folgendes umfasst:

einen Hauptkörper (10) für den Empfang des Objekts (14);
eine Einrichtung (16, 18, 20), um zu bestimmen, wann sich das Objekt korrekt an dem Körper befindet;

wobei es sich bei dem sendenden piezoelektrischen ersten Messwandler (28) um einen piezoelektrischen Ultraschallsender handelt, um ein Signal durch das Objekt zu senden, wobei der genannte Sender einen ersten hohlen Behälter (32) umfasst, der verschiebbar in dem Hauptkörper (10) aufgenommen wird, und wobei ein piezoelektrischer Keramiksender (34) federnd in dem genannten ersten Behälter (32) angebracht ist, wobei der genannte Sender einen Durchmesser aufweist, der etwa fünfmal so groß ist wie dessen Dicke;
eine Einrichtung (44, 60), welche den genannten Sender (28) in Richtung des genannten Objekts drängt;
eine Einrichtung (67, 69), um die Bewegung des genannten Senders (28) einzuschränken;
wobei es sich bei dem empfangenden piezoelektrischen zweiten Messwandler (46) um einen piezoelektrischen Ultraschallempfänger handelt, der räumlich getrennt von dem genannten Sender angeordnet ist und ferner in Richtung des genannten Körpers vorsteht, wobei der genannte Empfänger einen zweiten hohlen Behälter (52) umfasst, der verschiebbar in dem genannten Hauptkörper (10) aufgenommen wird, und mit einem keramischen Messwandler (48), der federnd in dem genannten zweiten hohlen Behälter (52) angebracht ist, wobei der genannte Messwandler einen Durchmesser aufweist, der mehr als fünfmal größer ist als dessen Dicke;
eine Einrichtung (38) zum Erzeugen eines zu übermittelnden Signals; und
eine Einrichtung (54) zur Verstärkung des empfangenen Signals.

**8.** Vorrichtung nach Anspruch 7, wobei es sich bei dem Objekt (14) um einen Fuß handelt.

**9.** Vorrichtung nach Anspruch 7, wobei die Einrichtung (16, 18, 20) zum Bestimmen, wann das Objekt korrekt positioniert ist, folgendes umfasst:

einen ersten Drucksensor (18) zur Berührung der Unterseite des Objekts; und
einen zweiten Drucksensor (16) zur Berührung der Rückseite des Objekts; und
eine Einrichtung (82) zum Erzeugen eines Signals, wenn Druck korrekt auf die ersten und zweiten Sensoren ausgeübt wird.

**10.** Vorrichtung nach Anspruch 9, wobei mindestens zwei erste Sensoren (18) gegeben sind.

**11.** Vorrichtung nach Anspruch 9, wobei wenn es sich bei dem Objekt (14) um einen Fuß handelt, die Einrichtung (16, 18, 20) zur Bestimmung, wann das Objekt korrekt positioniert ist, einen Zehenstabilisator (20) aufweist, der in der genannten Aussparung angebracht ist, so dass er an einer Seite des Zehs anstößt, um sicherzustellen, dass sich der Fuß an der korrekten Position befindet.

**12.** Vorrichtung nach Anspruch 11, wobei der Zehenstabilisator (20) für Füße unterschiedlicher Größe abgestuft ist.

**13.** Vorrichtung nach Anspruch 11, wobei der Zehenstabilisator (20) umkehrbar ist, so dass er sowohl in Verbindung mit dem linken als auch mit dem rechten Fuß verwendet werden kann.

**14.** Vorrichtung nach Anspruch 7, wobei der genannte Ultraschallsender (28) eine Einrichtung (100) zur Verstärkung eines Impulses aufweist.

**15.** Vorrichtung nach Anspruch 7, welche O-Ringe (40, 49) an den genannten ersten und zweiten hohlen Behältern aufweist, um es zu ermöglichen, dass der hohle Behälter in dem genannten Hauptkörper (10) verschoben wird.

**16.** Vorrichtung nach Anspruch 7, wobei Keramikmesswandler (34, 48) an einer federnden Endplatte (36, 50) der genannten ersten und zweiten Körper (32, 52) angebracht sind.

17. Vorrichtung nach Anspruch 16, wobei die genannten Endplatten (36, 50) aus einem federnden Klebstoff auf Styrolbasis gebildet werden.

18. Vorrichtung nach Anspruch 7, wobei die Einrichtung (44, 60), die den genannten Sender (28) in die genannte Vertiefung drückt, eine Feder (60) umfasst, die in einem dritten Behälter (44) angebracht ist, angebracht an dem ersten Behälter.

19. Vorrichtung nach Anspruch 18, wobei die Feder (60) an einem Ende des genannten dritten Behälters (32) anstößt; mit einem Kolben (64), der an die genannte Feder anstößt, um die genannte Feder zusammenzudrücken; mit einer Einrichtung (67, 69) zur Fixierung der Position des Kolbens (64) und somit der Spannung der Feder (60).

20. Vorrichtung nach Anspruch 19, wobei der Kolben (64) an einer Stange (66) angebracht ist, die sich aus dem genannten dritten Behälter (44) und aus dem genannten Hauptkörper (10) erstreckt; mit einem Kragen (65) an dem genannten IIauptkörper, mit ein ersten Eingriffseinrichtung (68); mit einer zweiten Eingriffseinrichtung (70) an der genannten Stange (66), eingriffsfähig mit der genannten ersten Eingriffseinrichtung (68), wobei die Rotation der Stange (66) so wirkt, dass die genannten ersten und zweiten Eingriffseinrichtungen freigegeben werden und miteinander eingreifen.

21. Verfahren zum Messen der physikalischen Eigenschaften eines Objekts, wobei das Verfahren folgendes umfasst:

das Empfangen von Energie von einem sendenden ersten piezoelektrischen Messwandler in einem zweiten empfangenden piezoelektrischen Messwandler, wobei der erste piezoelektrische Messwandler und der zweite piezoelektrische Messwandler räumlich getrennt voneinander sind, um das Objekt dazwischen zu empfangen; wobei das Verfahren **dadurch gekennzeichnet ist, dass** die genannte empfangene Energie von einem sendenden ersten piezoelektrischen Messwandler in einem zweiten empfangenden piezoelektrischen Messwandler das Empfangen von Energie von einem sendenden ersten piezoelektrischen Messwandler in einem zweiten empfangenden piezoelektrischen Messwandler umfasst, wobei die ersten (34, 48) und zweiten piezoelektrischen Messwandler jeweils durch eine Front an einer federnden Frontplatte (36, 50) angebracht sind, wobei jeder der piezoelektrischen Messwandler in der Lage ist, nach dem Empfang eines Energieimpulses radial und axial in Resonanz zu treten.

22. Verfahren nach Anspruch 21, wobei das Verfahren es umfasst, dass das Objekt Resonanzfrequenzen von dem ersten piezoelektrischen Ultraschallsender ausgesetzt wird, wobei eine erste Resonanzfrequenz erhalten wird durch in Resonanz treten in dem Dickemodus, und wobei eine zweite, niedrigere Resonanzfrequenz erhalten wird durch in Resonanz treten in dem radialen Modus.

23. Verfahren nach Anspruch 22, wobei das Verfahren das Bestimmen der optimalen hohen und niedrigen Frequenzen umfasst, indem eine niedrige Frequenzkomponente mit schwacher Dämpfung beim Verlauf durch das Objekt gefunden wird, und indem eine hohe Frequenzkomponente mit hoher Dämpfung beim Verlauf durch das Objekt gefunden wird.

24. Verfahren nach Anspruch 21, wobei das Verfahren das folgendes umfasst:

das Aussetzen des Objekts eines Hochfrequenzbündels unter Verwendung sowohl einer radialen Modus- als auch einer Dickenmodusresonanz eines piezoelektrischen Messwandlersenders; das Messen der durch das genannte Hochfrequenzbündel durch das Objekt übermittelten Energie; und das Ausdrücken der Energie als prozentualen Anteil der insgesamt empfangenen Energie, wobei dies folgendes umfasst:

(i) das Aufzeichnen der von dem Empfänger ausgegebenen Spannungsspur;
(ii) das Analysieren der Spur durch Spektralanalyse oder Differenzspur;
(iii) das Ableiten eines Ausdrucks:

$$\frac{[\text{Bereich der Hochfrequenzkomponente}]}{[\text{Bereich der gesamten Spur}]} \quad \begin{array}{l} \text{x } 100 \\ \\ - 100 \end{array}$$

wobei der genannte Ausdruck die prozentuale Übertragung auf die höhere Frequenzkomponente umfasst, ohne dass eine Referenz verwendet werden muss.

25. Verfahren nach Anspruch 24 zum Ableiten eines Index de strukturellen Integrität von Knochen unter Verwendung des in Anspruch 25 definierten Ausdrucks, wobei ein hoher Prozentwert eine gute Struktur anzeigt, und wobei Werte unter 80% progressiv schlechteren Knochen anzeigen.

26. Verfahren nach Anspruch 21, wobei es sich bei dem Objekt um Knochen handelt, und wobei das Verfahren folgendes umfasst:

(i) das Messen der Knochendichte unter Verwendung radialer und Dickenmodi der Resonanz eines piezoelektrischen Messwandlersenders, ohne dass eine Referenz verwendet wird, indem Fehler durch Variabilität in dem Kopplungsmedium eliminiert werden;

(ii) das Bestimmen der Mindestspannung eines Niederfrequenzbündels, das eine zu vernachlässigende Dämpfung beim Verlauf durch den Knochen anzeigt, um die Amplitude zu erreichen, die für zukünftige Messungen zu verwenden ist unter Verwendung einer großen Anzahl von Messungen an einem normalen Subjekt, um die Mindestspannung zu ermitteln, wobei die genannte Mindestspannung einen Normalisierungsfaktor (LFNF) darstellt, der bei allen folgenden Messungen aller Subjekte gemäß der folgenden Beziehung verwendet wird:

$$\text{Dichte} = \frac{\text{LFNF (Spannung)}}{\text{Niedrige Frequenz (Spannung)}} \quad \text{x} \quad \frac{\text{Hohe Frequenz (Spannung)}}{\text{Fersenbreite (mm)}}$$

$$= \frac{\text{Spannung}}{\text{mm}}$$

(iii) wobei die als Volt/mm ausgedrückte Spannung an einer großen Anzahl von jungen Erwachsenen gemäß dem folgenden Ausdruck bestimmt wird:

$$\text{Dichte} = \frac{[\text{Patient (Volt/mm) x 100}]}{[\text{normale junge Erwachsene (Volt/mm)}]}$$

wodurch Dichte = ein prozentualer Anteil normaler junger Erwachsener ist.

27. Verfahren nach Anspruch 21, wobei das Verfahren folgendes umfasst:

den Einsatz von radialen und Dicken-Resonanzmodi eines piezoelektrischen Messwandlers, um Fehler aufgrund eines Kopplungsmediums zu verhindern, indem ein Impuls mit niedriger Frequenz und zu vernachlässigender Dämpfung durch das Objekt übertragen wird, so dass eine gewünschte feste Spannung an dem Empfänger erhalten wird;

das Wiederholen dieser Messungen an der gleichen Person über einen langen Zeitraum unter Verwendung der durchgängig gleichen Kopplung und Aufzeichnung der niedrigsten benötigten Spannung an dem Sender/Messwandler, um die feste Spannung zu erreichen;

das Verwenden dieser Frequenzspannung als Niederfrequenz-Normalisierungsfaktor (LFNF);

das Senden eines Niederfrequenzbündels gefolgt von einem Hochfrequenzbündel; und

das Ausdrücken des LFNF als Zähler und der niedrigen Frequenz als Nenner, um die Hochfrequenzkomponente zu normalisieren.

28. Verfahren nach Anspruch 21, wobei das Verfahren das Messen der relativen Energie eines Hochfrequenzbündels misst, das durch das Objekt übertragen wird, durch:

das Aufzeichnen der empfangenen Energie unter Verwendung eines piezoelektrischen keramischen Messwandlers mit zwei Betriebsmodi, einem Dicken-Betriebsmodus und einem radialen Betriebsmodus;

das Ausführen einer Spektralanalyse einer Spur der empfangenen Energie;

das Bestimmen des Bereichs der Hochfrequenzkomponente; und

das Vergleichen des Bereichs mit dem durch ein Standardobjekt mit bekannten Eigenschaften übertragenen Bereichs, um Unterschiede in Bezug auf die Zusammensetzung oder die Struktur zwischen dem Objekt und dem Standard zu zeigen.

29. Verfahren nach Anspruch 28, wobei die Elastizitätskonstante des keramischen Messwandlers eingesetzt wird, um eine externe Referenz zum Messen der empfangenen Energie überflüssig zu machen.

30. Verfahren nach Anspruch 21, wobei das Verfahren das Messen der Verlaufszeit einer Schallwelle durch das Objekt umfasst, wobei es sich bei dem sendenden Messwandler um einen keramischen Ultraschallmesswandler handelt, der in den radialen und Dicken-Modi arbeitet, durch:

das Verstärken einer Spur der Schallwelle;

das Messen der Zeit des ersten Kreuzens der Basislinie einer Spur der empfangenen Schallwelle;

das Messen einer großen Anzahl ähnlicher Objekte, um einen Durchschnittswert abzuleiten und somit ein konstantes Zeitintervall;

das Einfügen dieses Durchschnittswerts in einen Mikroprozessor für eine Subtraktion von der Zeit der zweiten Kreuzung der Basislinie gemäß der Bestimmung durch den Mikroprozessor; und

**dadurch** das Erhalten der Verlaufszeit der Schallwelle durch ein Objekt.

31. Verfahren nach Anspruch 30, wobei die Zeitkonstante bei 668.400 Hz als 2,026 Mikrosekunden durch das menschliche Fersenbein bestimmt ist.

**Revendications**

1. Appareil pour mesurer les caractéristiques physiques d'un objet, comprenant un premier transducteur piézoélectrique émetteur (28; 34) et un second transducteur piézoélectrique récepteur (46; 48) pour recevoir de l'énergie du premier transducteur, lesdits premier (28; 34) transducteur et second (46; 48) transducteur étant espacés l'un de l'autre pour recevoir l'objet entre eux;
l'appareil étant **caractérisé en ce que** les premier (34) et second (48) transducteurs sont montés chacun sur une plaque frontale élastique (36, 50) par une face, moyennant quoi chacun des transducteurs peut résonner radialement et axialement lors de la réception d'une impulsion d'énergie.

2. Appareil selon la revendication 1, comprenant des moyens (38; 82, 96, 100) pour appliquer une tension au premier transducteur (34; 28) et des moyens (54, 56, 58; 82, 89, 102, 104) pour mesurer l'énergie dans le second transducteur (48; 46).

3. Appareil selon la revendication 1, dans lequel les premier (34) et second (48) transducteurs sont montés chacun à l'intérieur d'un tube (32, 52) ayant une plaque frontale (36, 50) au niveau de son extrémité.

4. Appareil selon la revendication 3, dans lequel chaque plaque frontale (36, 50) est moulée selon une forme prédéterminée (122) pour concentrer un rayonnement.

5. Appareil selon la revendication 1, dans lequel la plaque frontale élastique (36, 50) présente les caractéristiques suivantes:

(i) une forte adhérence à la face de la céramique et au corps principal cylindrique;

(ii) une impédance acoustique entre celles d'une céramique et d'un tissu humain;

(iii) une inertie face aux produits caustiques, aux acides et aux huiles;

(iv) une aptitude au moulage; et

(v) aucune conductivité électrique.

6. Appareil selon la revendication 5, dans lequel le matériau élastique est un polymère à base de styrène.

7. Appareil selon la revendication 1, comprenant en outre:

   un corps principal (10) pour recevoir l'objet (14);
   des moyens (16, 18, 20) pour déterminer le moment où l'objet est placé correctement sur le corps;
   le premier transducteur piézoélectrique émetteur (28) étant un émetteur à ultrason piézoélectrique destiné à envoyer un signal à travers l'objet, ledit émetteur comprenant un premier récipient creux (32) qui est reçu de manière coulissante dans le corps principal (10) et un émetteur en céramique piézoélectrique (34) monté de manière élastique dans ledit premier récipient (32), ledit émetteur ayant un diamètre environ 5 fois supérieur à son épaisseur;
   des moyens (44, 60) pressant ledit émetteur (28) vers ledit objet;
   des moyens (67, 69) pour limiter le mouvement dudit émetteur (28);
   le second transducteur piézoélectrique récepteur (46) étant un récepteur à ultrason piézoélectrique espacé dudit émetteur et faisant également saillie vers ledit corps, ledit récepteur comprenant un deuxième récipient creux (52) reçu de manière coulissante dans ledit corps principal (10), et un transducteur en céramique (48) monté de manière élastique dans ledit deuxième récipient creux (52), ledit transducteur ayant un diamètre plus de 5 fois supérieur à son épaisseur;
   des moyens (38) pour produire un signal devant être transmis; et
   des moyens (54) pour amplifier ledit signal reçu.

8. Appareil selon la revendication 7, dans lequel l'objet (14) est un pied.

9. Appareil selon la revendication 7, dans lequel les moyens (16, 18, 20) pour déterminer le moment où l'objet est correctement placé comprennent:

   un premier capteur de pression (18) destiné à être en contact avec le côté inférieur de l'objet; et
   un second capteur de pression (16) destiné à être en contact avec l'arrière de l'objet; et
   des moyens (82) pour produire un signal lorsqu'une pression est appliquée correctement aux premier et second capteurs.

10. Appareil selon la revendication 9, dans lequel il existe deux premiers capteurs (18).

11. Appareil selon la revendication 9 dans lequel, lorsque l'objet (14) est un pied, les moyens (16, 18, 20) pour déterminer le moment où l'objet est placé correctement comprennent un stabilisateur d'orteil (20) monté dans ledit évidement pour venir en butée contre un côté de l'orteil afin d'assurer que le pied est placé correctement.

12. Appareil selon la revendication 11, dans lequel le stabilisateur d'orteil (20) est comporte des crans correspondant à des pointures variables.

13. Appareil selon la revendication 11, dans lequel le stabilisateur d'orteil (20) est réversible afin de pouvoir servir à la fois pour le pied gauche et pour le pied droit.

14. Appareil selon la revendication 7, dans lequel ledit émetteur à ultrason (28) comprend des moyens (100) pour amplifier une impulsion.

15. Appareil selon la revendication 7, comprenant des joints toriques (40, 49) sur lesdits premier et deuxième récipients creux pour permettre au récipient creux de coulisser dans ledit corps principal (10).

16. Appareil selon la revendication 7, dans lequel les transducteurs en céramique (34, 48) sont montés sur une plaque d'extrémité élastique (36, 50) desdits premier et second corps (32, 52).

17. Appareil selon la revendication 16, dans lequel lesdites plaques d'extrémité (36, 50) sont formées d'un adhésif élastique à base de styrène.

18. Appareil selon la revendication 7, dans lequel les moyens (44, 60) pressant ledit émetteur (28) dans ledit évidement comprennent un ressort (60) monté dans un troisième récipient (44), fixé au premier récipient.

**19.** Appareil selon la revendication 18, dans lequel le ressort (60) est en butée contre une extrémité dudit troisième récipient (32);
un piston (64) venant en butée contre ledit ressort pour comprimer ledit ressort;
des moyens (67, 69) fixant la position du piston (64) et donc la tension du ressort (60).

**20.** Appareil selon la revendication 19, dans lequel le piston (64) est monté sur une tige (66) qui s'étend à l'extérieur dudit troisième récipient (44) et à l'extérieur dudit corps principal (10);
un collier (65) sur ledit corps principal ayant des premiers moyens de mise en prise (68);
des seconds moyens de mise en prise (70) sur ladite tige (66), pouvant se mettre en prise avec lesdits premiers moyens de mise en prise (68) moyennant quoi la rotation de la tige (66) entraîne la libération et la mise en prise desdits premiers et seconds moyens de mise en prise.

**21.** Procédé pour mesurer les caractéristiques physiques d'un objet, consistant à:

recevoir de l'énergie d'un premier transducteur piézoélectrique émetteur dans un second transducteur piézoélectrique récepteur, le premier transducteur piézoélectrique et le second transducteur piézoélectrique étant espacés l'un de l'autre pour recevoir l'objet entre eux;
le procédé étant **caractérisé en ce que** ladite réception d'énergie d'un premier transducteur piézoélectrique émetteur dans un second transducteur piézoélectrique récepteur comprend une réception d'énergie d'un premier transducteur piézoélectrique émetteur dans un second transducteur piézoélectrique récepteur, dans laquelle les premier (34, 48) et second transducteurs piézoélectriques sont montés chacun sur une plaque frontale élastique (36, 50) par une face, moyennant quoi chacun des transducteurs piézoélectriques peut résonner radialement et axialement lors de la réception d'une impulsion d'énergie.

**22.** Procédé selon la revendication 21, consistant à soumettre l'objet à des fréquences de résonance émanant du premier émetteur à ultrason piézoélectrique, une première fréquence de résonance obtenue par résonance dans le mode selon l'épaisseur et une seconde fréquence de résonance, plus basse, obtenue par résonance dans le mode radial.

**23.** Procédé selon la revendication 22, qui consiste à déterminer les fréquences haute et basse optimales en obtenant une composante basse fréquence ayant une faible atténuation lors du passage à travers l'objet et en obtenant une composante haute fréquence ayant une forte atténuation lors du passage à travers l'objet.

**24.** Procédé selon la revendication 21, consistant à:

soumettre l'objet à une rafale haute fréquence en utilisant un mode de résonance à la fois radial et selon l'épaisseur d'un émetteur transducteur piézoélectrique;
mesurer l'énergie transmise à travers l'objet par ladite rafale haute fréquence; et
exprimer cette énergie sous forme d'un pourcentage d'une énergie totale reçue, ce qui consiste à:

(i) enregistrer la trace de tension produite par le récepteur;
(ii) analyser la trace par analyse spectrale ou trace différentielle;
(iii) calculer une expression:

$$\frac{[aire.de.la.composante.haute.fréquence] \times 100}{[aire.de.la.trace.complète] - 100}$$

ladite expression déterminant la transmission en pourcentage vers la composante de plus haute fréquence sans qu'il soit nécessaire d'utiliser une référence.

**25.** Procédé selon la revendication 24 pour calculer un indice d'intégrité structurelle d'un os en utilisant l'expression définie dans la revendication 25, un pourcentage élevé indiquant une bonne structure et des valeurs inférieures à 80 % indiquant un os dont la qualité diminue progressivement.

**26.** Procédé selon la revendication 21, dans lequel l'objet est un os, et qui consiste à:

(i) mesurer la densité osseuse en utilisant les modes de résonance radial et selon l'épaisseur d'un émetteur transducteur piézoélectrique sans utiliser de référence en éliminant l'erreur liée à la variabilité du milieu de couplage,

(ii) déterminer la tension minimale d'une rafale basse fréquence présentant une atténuation négligeable lors du passage à travers l'os pour obtenir l'amplitude à utiliser pour les mesures futures en utilisant un grand nombre de mesures sur un sujet normal pour trouver la tension minimale, ladite tension minimale étant un facteur de normalisation (LFNF) utilisé sur toutes les mesures ultérieures de tous les sujets selon la relation suivante:

$$\text{Densité} = \frac{\text{LFNF(tension)}}{\text{basse.fréquence(tension)}} \times \frac{\text{haute.fréquence(tension)}}{\text{largeur.de.talon(mm)}}$$
$$= \frac{\text{tension}}{\text{mm}}$$

(iii) la densité exprimée sous forme de volts/mm étant déterminée sur un nombre élevé de jeunes adultes selon l'expression suivante:

$$\text{Densité} = \frac{\left[\text{patient(volt/mm) x 100}\right]}{\left[\text{jeunes.adultes.normaux(volts/mm)}\right]}$$

moyennant quoi la densité est un pourcentage de jeunes adultes normaux.

27. Procédé selon la revendication 21, consistant à:

utiliser les modes de résonance radial et selon l'épaisseur d'un transducteur piézoélectrique pour éliminer une erreur provoquée par un milieu de couplage en transmettant une impulsion de basse fréquence et d'atténuation négligeable à travers l'objet pour obtenir une tension fixe souhaitée au niveau du récepteur;
répéter ces mesures sur le même individu pendant une longue période en utilisant le même couplage du début à la fin et en enregistrant la plus basse tension nécessaire au niveau de l'émetteur/transducteur pour obtenir la tension fixe;
utiliser cette tension de fréquence comme un facteur de normalisation basse fréquence (LFNF);
envoyer une rafale basse fréquence suivie d'une rafale haute fréquence; et
exprimer le LFNF comme numérateur et la basse fréquence comme dénominateur pour normaliser la composante haute fréquence.

28. Procédé selon la revendication 21, consistant à mesurer l'énergie relative d'une rafale haute fréquence transmise à travers l'objet de la manière suivante:

en enregistrant l'énergie reçue au moyen d'un transducteur en céramique piézoélectrique ayant deux modes de fonctionnement, à savoir un mode de fonctionnement selon l'épaisseur et un mode de fonctionnement radial;
en effectuant une analyse spectrale d'une trace de l'énergie reçue;
en déterminant l'aire de la composante haute fréquence; et
en comparant l'aire avec celle transmise à travers un objet normal de caractéristiques connues, pour montrer les différences de composition ou de structure entre l'objet et la norme.

29. Procédé selon la revendication 28, dans lequel le module d'élasticité du transducteur en céramique permet de ne plus avoir besoin d'une référence externe pour mesurer l'énergie reçue.

30. Procédé selon la revendication 21, consistant à mesurer le temps de parcours d'une onde sonore à travers l'objet, dans lequel le transducteur émetteur est un transducteur à ultrason en céramique piézoélectrique très faiblement atténué fonctionnant dans des modes radial et selon l'épaisseur, de la manière suivante:

en amplifiant une trace de l'onde sonore;
en mesurant l'instant du premier franchissement de la ligne de base d'une trace de l'onde sonore reçue;
en mesurant un nombre élevé d'objets similaires pour calculer une valeur moyenne et donc un intervalle de temps constant;

en introduisant cette valeur moyenne dans un microprocesseur pour la soustraire de l'instant du deuxième franchissement de la ligne de base tel que déterminé par le microprocesseur; et

en obtenant ainsi le temps de parcours de l'onde sonore à travers un objet.

31. Procédé selon la revendication 30, dans lequel la constante de temps est déterminée à 668 400 Hz comme étant égale à 2,026 microsecondes pour l'os du talon humain.

FIG. 1

FIG. 1a

FIG. 2

FIG. 4a

FIG. 4b

FIG. 3a

FIG. 5

FIG. 3b

FIG. 6

FIG. 7a

FIG. 7b

FIG. 7c

**EP 1 173 097 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 018709 A